(19) Europäisches Patentamt
European Patent Office
Office européen des brevets

(11) Numéro de publication: **0 155 888**
**A1**

# (12) DEMANDE DE BREVET EUROPEEN

(21) Numéro de dépôt: **85400437.1**

(22) Date de dépôt: **07.03.85**

(51) Int. Cl.⁴: **C 07 D 401/06**
**A 61 K 31/47**

(30) Priorité: **09.03.84 FR 8403669**

(43) Date de publication de la demande:
**25.09.85 Bulletin 85/39**

(84) Etats contractants désignés:
**AT BE CH DE FR GB IT LI LU NL SE**

(71) Demandeur: **RHONE-POULENC SANTE**
**Les Miroirs 18 Avenue d'Alsace**
**F-92400 Courbevoie Cedex(FR)**

(72) Inventeur: **Renault, Christian**
**61 rue des Mallets**
**F-95150 Taverny(FR)**

(72) Inventeur: **Mestre, Michel**
**1 rue Scheffer**
**F-75116 Paris(FR)**

(74) Mandataire: **Gaumont, Robert et al,**
**RHONE-POULENC RECHERCHES Service Brevets**
**Pharma 25, Quai Paul Doumer**
**F-92408 Courbevoie Cedex(FR)**

(54) **Dérivés de (quinol yl-4)-1 (piperidyl-4)-2 ethanamine et de (quinolyl-4)-1 (piperidyl-4)-3 propanamine, procédés pour leur préparation et médicaments les contenant.**

(57) La présente invention a pour objet des dérivés de formule (I) dans laquelle n est égal à 1 ou 2.

R représente l'atome d'hydrogène, un groupe alkyle ayant 1 à 4 atomes de carbone ou le radical phényle,

$R_1$ représente l'atome d'hydrogène, un groupe alkyle comportant 1 à 4 atomes de carbone ou un groupe alcényle ayant 2 à 4 atomes de carbone,

X et Y, identiques ou différents, sont fixés en position 5,6,7 ou 8 sur le cycle de la quinoléine et représentent l'atome d'hydrogène ou un groupe alcoxy comportant 1 à 3 atomes de carbone,

leurs procédés de préparation et les médicaments les contenant.

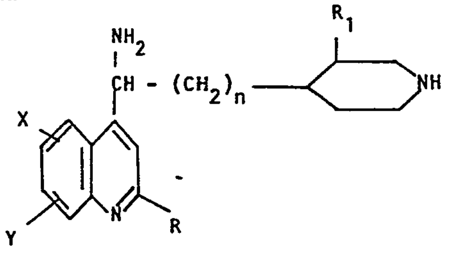

(I)

EP 0 155 888 A1

La présente invention a pour objet de nouveaux dérivés de la (quinolyl-4)-1 (pipéridyl-4)-2 éthanamine et de la (quinolyl-4)-1 (pipéridyl-4)-3 propanamine utilisables comme médicaments et leurs procédés de préparation.

Ces dérivés peuvent être représentés par la formule générale (I) dans laquelle n est égal à 1 ou 2,
R représente l'atome d'hydrogène, un groupe alkyle ayant 1 à 4 atomes de carbone ou le radical phényle,
$R_1$ représente l'atome d'hydrogène, un groupe alkyle comportant 1 à 4 atomes de carbone ou un groupe alcényle ayant 2 à 4 atomes de carbone,
X et Y, identiques ou différents, sont fixés en position 5,6,7 ou 8 sur le cycle de la quinoléine et représentent l'atome d'hydrogène ou un groupe alcoxy comportant 1 à 3 atomes de carbone.

Les composés préférés sont ceux dans lesquels Y est l'atome d'hydrogène, X l'atome d'hydrogène ou le groupe méthoxy, R l'atome d'hydrogène, le groupe tertiobutyle ou le radical phényle et $R_1$ l'atome d'hydrogène, ou le groupe éthényle ou éthyle.

Lorsque $R_1$ représente l'atome d'hydrogène, la molécule des composés de formule (I) comporte un atome de carbone asymétrique (atome de carbone portant le groupe amino) et donc, pour une signification donnée de X, Y, R, $R_1$ et n il y a un racémique et deux énantiomères.

Lorsque $R_1$ ne représente pas l'atome d'hydrogène, la molécule des composés de formule (I) comporte 3 atomes de carbone asymétriques et donc, pour une signification donnée de X, Y, R, $R_1$ et n il y a 8 stéréoisomères dont les formules spatiales correspondent aux combinaisons 3 à 3 des configurations rectus (R) ou sinister (S) de chaque centre d'asymétrie.

Les divers isomères signalés ci-dessus font partie de l'invention de même que les sels d'addition des composés répondant à la formule générale (I) avec des acides minéraux ou organiques.

Les composés de formule générale (I) peuvent être préparés à partir des cétones de formule (II) dans laquelle n, X, Y, R et $R_1$ ont les mêmes significations que dans la formule (I), par des

procédés qui permettent de transformer une cétone en amine primaire comme ceux, par exemple, décrits dans C.A. BUEHLER et D.E. PEARSON, Survey of Organic Synthesis, Wiley Interscience, vol. 1, 1970, p. 423 et 427.

Une méthode avantageuse consiste en un traitement de la cétone de formule (II) par le formiate d'ammonium à une température variant de 150 à 200°C suivi d'une hydrolyse en milieu acide aqueux au reflux.

Une autre méthode consiste à traiter la cétone de formule (II) avec de l'hydroxylamine au sein d'un solvant tel que l'éthanol à une température variant de la température ambiante à la température d'ébullition du solvant puis à réduire l'oxime précédemment obtenue par des procédés tels que ceux décrits dans HOUBEN-WEYL, Methoden der Organischen Chemie, 11 (1) p. 495 (Georg. Thieme Verlag - Stuttgart, 1957).

Une méthode particulièrement avantageuse de réduction de l'oxime consiste à utiliser la poudre de zinc en milieu ammoniacal hydroalcoolique.

Lorsque $R_1$ est l'atome d'hydrogène, les cétones de formule (II) conduisent à des composés de formule (I) racémiques.

Lorsque $R_1$ n'est pas un atome d'hydrogène, les cétones de formule (II) conduisent à un mélange de composés diastéréoisomères racémiques ou optiquement actifs selon que la cétone de départ est racémique ou optiquement active.

Les composés de formule (I) dans laquelle $R_1$ représente un groupe alkyle comportant 2 à 4 atomes de carbone peuvent aussi être préparés par hydrogénation catalytique des composés correspondants de formule (I) dans laquelle $R_1$ représente un groupe alcényle ayant 2 à 4 atomes de carbone. Cette hydrogénation peut par exemple être réalisée à température ambiante, sous une pression d'hydrogène égale à la pression atmosphérique, au sein d'un solvant inerte tel qu'un alcool, par exemple méthanol ou éthanol, en présence d'un catalyseur tel que le palladium, le nickel, le rhodium, le ruthénium ou le platine.

Les diastéréoisomères purs peuvent être isolés à partir du mélange par des méthodes classiques telles que chromatographie, cristallisation fractionnée, formation de sels et régénération de base.

Les cétones de formule (II) peuvent être préparées par le procédé décrit dans le brevet français 2 495 470.

Les exemples suivants illustrent l'invention sans la limiter.

EXEMPLE 1 : [ETHENYL-3 (R) PIPERIDYL-4 (R)]-3 (METHOXY-6 QUINOLYL-4)-1 PROPANAMINE-1 (RS).

On porte 24 h au reflux un mélange de 21 g de quinicine, 5,5 g de chlorhydrate d'hydroxylamine et 6,4 g d'acétate de sodium anhydre dans 260 ml d'éthanol.

On évapore l'éthanol, reprend le résidu à l'eau,alcalinise la solution aqueuse au moyen d'ammoniaque et extrait l'insoluble à l'acétate d'éthyle.

On lave la phase organique à l'eau, la sèche sur du sulfate de magnésium anhydre et l'évapore à sec sous pression réduite. On obtient 21 g de produit que l'on reprend dans 125 ml d'éthanol, 125 ml d'eau et 19 ml d'ammoniaque concentré auxquels on ajoute 5 g d'acétate d'ammonium. On agite à la température ambiante et ajoute par fractions 19 g de zinc en poudre. Enfin on porte le mélange au reflux pendant 3 heures.

Après refroidissement on filtre le mélange sur Clarcel et élimine l'éthanol sous pression réduite. On reprend le résidu à l'eau, alcalinise la phase aqueuse au moyen d'hydroxyde de sodium et extrait l'huile qui relargue au chloroforme. On lave la phase organique à l'eau, la sèche et l'évapore à sec sous pression réduite On obtient 20 g de produit que l'on chromatographie sur gel de silice au moyen d'un mélange éthanol-diéthylamine (99 : 1) comme éluant. On obtient 16 g de produit que l'on traite par 2,81 g d'acide furamique au sein de l'éthanol. Après recristallisation dans le même solvant on obtient 5,2 g d'[éthényl-3 (R) pipéridyl-4 (R)]-3 (méthoxy-6 quinolyl-4)-1 propanamine-1 (R,S) sous forme de sesquifumarate fondant à 220°C.

EXEMPLE 2 [[(DIMETHYL-1,1 ETHYL)-2 QUINOLYL]-4]-1 (PIPERIDYL-4)-3 PROPANAMINE-1

A 6 g de [[(diméthyl-1,1 éthyl)-2 quinolyl]-4]-1 (pipéridyl-4)-3 propanone-1 on ajoute 15 g de formiate d'ammonium et porte le mélange des réactifs 8 heures à 160°C. Après refroidissement on reprend le mélange avec du chlorure de méthylène et de l'eau. On lave la phase organique à l'eau, la sèche et l'évapore à sec sous pression réduite. On reprend le résidu dans 60 ml d'acide chlorhydrique 6N et porte le mélange 22 heures au reflux.

On dilue à l'eau, alcalinise la solution avec une lessive concentrée de soude et extrait l'insoluble avec du chlorure de méthylène. On lave la phase organique à l'eau, la sèche sur du sulfate de magnésium anhydre et l'évapore à sec sous pression réduite. On obtient 4,1 g de produit que l'on transforme en dichlorhydrate au sein de l'éthanol. Après recristallisation dans l'éthanol à 95 % on obtient 1,55 g de dichlorhydrate de [[(diméthyl-1,1 éthyl)-2 quinolyl]-4]-1 (pipéridyl-4)-3 propanamine-1 fondant au-dessus de 265°C.

Le chlorhydrate de [[(diméthyl-1,1 éthyl)-2 quinolyl]-4]-1 (pipéridyl-4)-3 propanone-1 peut être préparé selon le brevet français 2 495 470.

EXEMPLE 3 [(PHENYL-2 QUINOLYL)-4]-1 (PIPERIDYL-4)-2 ETHANAMINE

On porte à 190°C pendant 18 heures un mélange de 11 g de (phényl-2 quinolyl-4)-1 (pipéridyl-4)-2 éthanone et de 27,3 g de formiate d'ammonium. Après refroidissement on reprend le milieu réactionnel dans du chloroforme et de l'eau. On lave la phase organique à l'eau, la sèche sur du sulfate de magnésium et l'évapore à sec sous pression réduite. On chromatographie le résidu sur du gel de silice avec un mélange chloroforme-diéthylamine (9:1) comme éluant. Après concentration des fractions recherchées on obtient 11,6 g de produit que l'on reprend dans 70 ml d'acide chlorhydrique 6N et que l'on porte à ébullition pendant 18 heures.

Après refroidissement on lave la phase aqueuse avec de l'éther éthylique, l'alcalinise avec une solution d'ammoniaque concentré et extrait l'insoluble au chloroforme. On lave la solution chloroformique à l'eau, la sèche sur du sulfate de magnésium et l'évapore à sec sous pression réduite. On obtient 8 g de produit que l'on chromatographie sur gel de silice avec un mélange chloroforme, méthanol, diéthylamine (8:1:1) comme éluant. Par concentration des fractions recherchées on obtient 4 g de produit que l'on traite par de l'éthanol chlorhydrique. On obtient ainsi 1,1 g de [(phényl-2 quinolyl)-4]-1 (pipéridyl-4)-2 éthanamine sous forme de dichlorhydrate fondant à 226°C.

La [(phényl-2 quinolyl)-4]-1 (pipéridyl-4)-2 éthanone peut être préparée selon le brevet français 2 471 981.

<u>EXEMPLE 4</u> [ETHYL-3(R) PIPERIDYL-4 (R)]-3 (METHOXY-6 QUINOLYL-4)-1 PROPANAMINE-1 (RS)

On hydrogène à la pression atmosphérique et à la température ambiante 1,2 g de [éthényl-3 (R) pipéridyl-4 (R)]-3 (méthoxy-6 quinolyl-4)-1 propanamine-1 (RS) en solution dans 25 ml d'éthanol absolu en présence de 0,25 g de palladium à 10 % sur charbon. L'absorption d'hydrogène est complète en 2 heures, on filtre le catalyseur, évapore le solvant sous pression réduite et recristallise le résidu dans de l'isopropanol. On obtient ainsi l'[éthyl-3 (R) pipéridyl-4 (R)]-3 (méthoxy-6 quinolyl-4)-1 propanamine-1 (RS) sous forme de trichlorhydrate fondant à 200°C. Le spectre de R.M.N. du proton, de la base, dans le chloroforme deutérié présente les caractéristiques suivantes :

$CH_3O$ - δ : 3,9 ppm, $H_2$ δ : 8,7 ppm,

$H_3$ δ : 7,5 ppm, $H_5$ δ : 7,3 ppm, $H_8$ δ : 8,0 ppm.

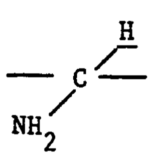 δ : 4,6 ppm, $-CH_2 - \underline{CH_3}$ δ : 0,8 ppm.

## PROPRIETES PHARMACOLOGIQUES

### Activité antiarythmique

L'activité antiarythmique des composés de formule (I) a été démontrée à l'aide du test de l'aconitine.

Le principe de la technique repose sur le temps d'induction des arythmies ventriculaires provoquées par l'aconitine en perfusion lente chez le rat. Une substance antiarythmique retarde l'apparition des arythmies et ce délai est proportionnel à l'activité de la substance.

On utilise des groupes de 5 rats mâles. Une anesthésie individuelle est réalisée (uréthane 10 % : 1 g/kg/ip) pour permettre une cathétérisation de la veine du pénis. L'électrocardiogramme est enregistré. Au temps T = 0 la substance étudiée est injectée sous forme d'une solution aqueuse, à raison de 2,5 ml de solution par kg en 30 secondes. Au temps T = 60 secondes, soit 30 secondes après la fin de l'injection, l'aconitine est perfusée à raison de 20 µg par minute, jusqu'à l'apparition d'extra systoles supraventriculaires. Le temps de perfusion de l'aconitine est noté.

On exprime les résultats par une $DE_{50}$, dose de produit en mg/kg qui, par rapport aux animaux témoins, augmente de 50 % le temps de perfusion de l'aconitine.

Les résultats obtenus sont rassemblés dans le tableau ci-dessous :

| Produits | $DE_{50}$ mg/kg i.v. |
|---|---|
| Exemple 1 | 5 |
| Exemple 2 | 1,2 |
| Exemple 3 | 0,38 |
| QUINIDINE | 7,5 |

Les composés de formule (I) présentent de remarquables propriétés antiarythmiques et sont plus actifs que la quinidine.

## PROPRIETES TOXICOLOGIQUES

Les toxicités aiguës des composés selon l'invention ont été déterminées chez la souris mâle $CD_1$ (Charles RIVER) par la voie intraveineuse. Les $DL_{50}$ ont été calculées après 3 jours d'observation par la méthode cumulative de J.J. REED et H. MUENCH (Amer. J. Hyg. 1937, 27, 493).

Les $DL_{50}$ des composés de formule (I) sont supérieures à 15 mg/kg i.v.

## UTILISATION THERAPEUTIQUE

Les composés de l'invention et leurs sels pharmaceutiquement acceptables peuvent être utilisés en thérapeutique humaine pour le traitement et/ou la prévention des troubles du rythme. Ils peuvent se présenter sous toutes les formes en usage dans le domaine des médicaments telles que comprimés, capsules, gélules, suppositoires, solutions ingérables ou injectables.

La posologie dépend des effets recherchés et de la voie d'administration utilisée. Par exemple, par voie orale, elle peut être comprise entre 50 et 800 mg de substance active par 24 heures avec des doses unitaires variant de 10 à 100 mg de substance active.

## REVENDICATIONS

1 - Composés de formule (I) dans laquelle n est égal à 1 ou 2, R représente l'atome d'hydrogène, un groupe alkyle ayant 1 à 4 atomes de carbone ou le radical phényle, $R_1$ représente l'atome d'hydrogène, un groupe alkyle comportant 1 à 4 atomes de carbone ou un groupe alcényle ayant 2 à 4 atomes de carbone, X et Y, identiques ou différents, sont fixés en position 5,6,7 ou 8 sur le cycle de la quinoléine et représentent l'atome d'hydrogène ou un groupe alcoxy comportant 1 à 3 atomes de carbone ; leurs diastéréoisomères, racémiques et énantiomères et leurs sels d'addition avec des acides minéraux ou organiques.

2 - Composés selon la revendication 1 caractérisés en ce que Y représente l'atome d'hydrogène, X l'atome d'hydrogène ou le groupe méthoxy, R l'atome d'hydrogène, le groupe tertiobutyle ou le radical phényle et $R_1$ l'atome d'hydrogène ou le groupe éthényle ou éthyle.

3 - Procédé de prépration des composés selon les revendications 1 et 2 caractérisé en ce que l'on traite les cétones de formule (II) dans laquelle n, R, $R_1$, X et Y ont les mêmes significations que dans la formule (I)
par le formiate d'ammonium puis hydrolyse les produits obtenus.

4 - Procédé de préparation des composés selon les revendications 1 et 2 caractérisé en ce que l'on traite les cétones de formule (II) dans laquelle n, X, Y, R et $R_1$ ont les mêmes significations que dans la formule (I) avec l'hydroxylamine puis réduit l'oxime obtenue.

5 - Procédé de préparation des composés selon les revendications 1 et 2 pour lesquelles $R_1$ représente un groupe alkyle comportant 2 à 4 atomes de carbone par hydrogénation catalytique des composés correspondants pour lesquels $R_1$ représente un groupe alcényle ayant 2 à 4 atomes de carbone.

6 - Médicaments caractérisés en ce qu'ils contiennent comme substance active un composé tel que défini aux revendications 1 et 2 ou un sel d'un tel composé avec un acide pharmaceutiquement acceptable.

7 - Médicaments caractérisés en ce qu'il contiennent comme substance active la [(phényl-2 quinolyl-4]-1 pipéridyl-4)-2 éthanamine.

## REVENDICATION

1/ Procédé de préparation de composé de formule (I) dans laquelle n est égal à 1 ou 2, R représente l'atome d'hydrogène, un groupe alkyle ayant 1 à 4 atomes de carbone ou le radical phényle, $R_1$ représente l'atome d'hydrogène, un groupe alkyle comportant 1 à 4 atomes de carbone ou un groupe alcényle ayant 2 à 4 atomes de carbone, X et Y identiques ou différents sont fixés en position 5, 6, 7 ou 8 sur le cycle de la quinoléine et représentent l'atome d'hydrogène ou un groupe alcoxy comportant 1 à 3 atomes de carbone, leurs diastéréoisomères, racémiques et énantiomères et leurs sels d'addition avec des acides minéraux ou organiques,
caractérisé en ce que :

A - Dans le cas où n, R, $R_1$, X et Y ont toutes les significations précitées on traite une cétone de formule (II) dans laquelle n, R, $R_1$, X et Y ont les mêmes significations que dans la formule (I), par le formiate d'ammonium, hydrolyse le produit obtenu et transforme éventuellement en sel d'addition avec un acide ;

B - Dans le cas où n, R, $R_1$, X et Y ont toutes les significations précitées, on traite une cétone de formule (II) dans laquelle n, R, $R_1$, X et Y ont les mêmes significations que dans la formule (I), par l'hydroxylamine puis réduit l'oxime obtenue et transforme éventuellement en sel d'addition avec un acide ;

C - Dans le cas où n, R, X et Y ont toutes les significations précitées et $R_1$ représente un groupe alkyle comportant 2 à 4 atomes de carbone, on hydrogène catalytiquement le composé correspondant dans lequel $R_1$ représente un groupe alcényle ayant 2 à 4 atomes de carbone et transforme éventuellement en sel d'addition avec un acide.

PLANCHE 1/I

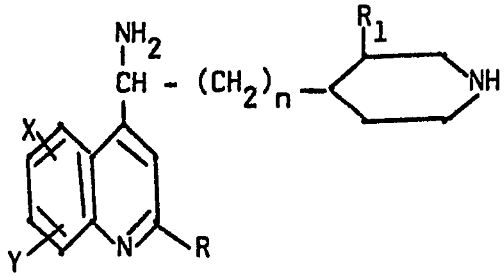

(I)

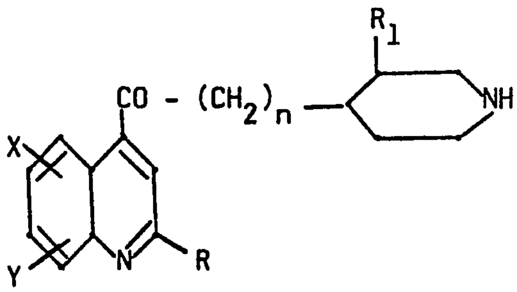

(II)

Office européen des brevets

# RAPPORT DE RECHERCHE EUROPEENNE

0155888

Numéro de la demande

EP 85 40 0437

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,A | EP-A-0 053 964 (PHARMINDUSTRIE)<br>* En entier *<br>--- | 1-7 | C 07 D 401/06<br>A 61 K 31/47 |
| A | EP-A-0 035 819 (ACF CHEMIEFARMA)<br>* En entier *<br>----- | 1-7 | |
| | | | DOMAINES TECHNIQUES RECHERCHES (Int. Cl.4) |
| | | | C 07 D 401/00 |

Le présent rapport de recherche a été établi pour toutes les revendications

| Lieu de la recherche | Date d'achèvement de la recherche | Examinateur |
|---|---|---|
| LA HAYE | 05-06-1985 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant